# EUROPEAN PATENT SPECIFICATION

(11) **EP 1 909 671 B1**
(45) Date of publication and mention of the grant of the patent: **18.01.2012**
(21) Application number: 06774646.1
(22) Date of filing: 11.07.2006
(51) Int. Cl.: A61B 17/88

(54) **SYSTEM FOR INSERTING BIOCOMPATIBLE FILLER MATERIALS IN INTERIOR BODY REGIONS**
SYSTEM ZUM EINFÜHREN VON BIOKOMPATIBLEN FÜLLMATERIALIEN IN INNERE KÖRPERREGIONEN
SYSTEME D'INSERTION DE MATIERES DE REMPLISSAGE DANS DES REGIONS CORPORELLES INTERIEURES

(30) Priority: 11.07.2005 US 698289 P
(43) Date of publication of application: 16.04.2008
(73) Proprietor: Kyphon SÀRL, 2000 Neuchâtel (CH)
(72) Inventor: EDIDIN, Avram, Allan, Portola Valley, CA 94028 (US)
(74) Representative: Desrousseaux, Grégoire Marie
(86) International application number: PCT/US2006/027049
(87) International publication number: WO 2007/008984

(56) References cited:
- WO-A-96/11643
- US-A- 4 969 888
- US-A1- 2001 049 531
- US-A1- 2002 161 373

## Description

### FIELD OF THE INVENTION

The invention relates to systems and methods for inserting biocompatible filler materials in interior body regions for diagnostic or therapeutic purposes.

### BACKGROUND

Certain diagnostic or therapeutic procedures require provision of a cavity in an interior body region. For example, as disclosed in U.S. Pat. Nos. 4,969,888 and 5,108,404, an expansible body may be deployed to form a cavity in cancellous bone tissue, as part of a therapeutic procedure that fixes fractures or other abnormal bone conditions, both osteoporotic and non-osteoporotic in origin. The expansible body may compress the cancellous bone to form an interior cavity in a treatment area. The cavity may receive a biocompatible filler material, such as a bone cement, which provides renewed interior structural support for cortical bone.

This procedure can be used to treat cortical bone, which due to osteoporosis, avascular necrosis, cancer, trauma, or other disease is fractured or is prone to compression fracture or collapse. These conditions, if not successfully treated, can result in deformities, chronic complications, and an overall adverse impact upon the quality of life. Certain biocompatible filler materials may chemically react with known expansible body devices, causing at least a part of the expansible body to weaken or dissolve, thereby permitting excess expansion in localized areas or even a gas or liquid material therewithin to flow into a treatment area.

WO 9611643 relates to a method for implanting a prosthetic spinal disc nucleus, in which there is described an inflatable jack used to spread apart the vertebrae adjacent to the implantation site.

Document US 2000/0156482 discloses a system for inserting biocompatible filler material in interior body regions according to the preamble of claim 1.

A demand exists for further systems and methods that are capable of inserting biocompatible filler materials in bone and other interior body regions in safe and efficacious ways.

### SUMMARY

The present invention provides a system as defined in claim 1. Embodiments of the present invention provide systems for inserting biocompatible filler materials in interior body regions. One illustrative embodiment comprises inserting an expansible body into a treatment area along a path established by a hollow member. The expansible body comprises body material substantially resistant to a biocompatible filler material or a chemical component thereof (e.g., a monomer). The expansible body may then be expanded within the treatment area, and the biocompatible filler material may be inserted into the treatment area while the expansible body is expanded. The biocompatible filler material may comprise, for example, a bone cement, and may be inserted into a treatment area to support a surrounding body structure, such as a vertebral body.

This embodiment is mentioned not to limit or define the invention, but to provide an example of an embodiment of the invention to aid understanding thereof. Illustrative embodiments are discussed in the Detailed Description, and further description of the invention is provided there. Advantages offered by the various embodiments of the present invention may be further understood by examining this specification.

### BRIEF DESCRIPTION OF THE FIGURES

These and other features, aspects, and advantages of the present invention are better understood when the following Detailed Description is read with reference to the accompanying drawings, wherein:
Figure 1 is a perspective view of a tool, wherein the expansible body thereof is shown in a collapsed state;
Figure 2 is a perspective view of the tool shown in Figure 1, wherein the expansible body thereof is shown in an expanded state;
Figure 3 is an elevation view of a system according to another tool;
Figure 4 is an elevation (lateral) view of several human vertebrae, with a hollow member establishing a percutaneous path to a vertebral body of one of the several vertebrae;
Figure 5 is a plan (coronal) view of a human vertebra being accessed by a system according to one embodiment of the present invention, with portions removed to reveal cancellous bone within the vertebral body;
Figure 6 is a plan (coronal) view of the vertebra and system shown in Figure 5, wherein a plurality of cavities has been provided within the vertebral body;
Figure 7 is a plan (coronal) view of the vertebra and system shown in Figures 5 and 6,
wherein a biocompatible filler material is shown being inserted into one of the cavities;
Figure 8 is a flow chart of a treatment method;
Figure 9 is a plan view of a sterile kit configured to store a single use tool according to one embodiment of the present invention; and
Figure 10 is an exploded perspective view of the sterile kit of Figure 9.

### DETAILED DESCRIPTION

Embodiments of the present invention provide systems for inserting biocompatible filler materials in interior body regions. The systems embodying the invention can be adapted for use in many suitable interior body regions, wherever the temporary or permanent formation, enlargement, adjustment, or maintenance of a cavity within or adjacent one or more layers of tissue may be required for a therapeutic or diagnostic purpose. The illustrative embodiments show the invention in association with systems and methods used to treat bones. In other embodiments, the present invention may be used in other interior body regions or types of tissues, such as intervertebral discs or cartilage.

As used in this specification and the appended claims, the singular forms "a," "an," and "the" include plural referents unless the context clearly dictates otherwise. Thus, for example, the term "a lumen" is intended to mean a single lumen or a combination of lumens. Furthermore, the words "proximal" and "distal" refer to directions closer to and away from, respectively, an operator (e.g., surgeon, physician, nurse, technician, etc.). An operator may insert a medical device into a patient, with at least a tip-end (i.e., distal end) of the device inserted inside a patient's body. Thus, in one example, the end of the medical device inserted inside the patient's body would be the distal end of the medical device, while the end of the medical device outside the patient's body would be the proximal end of the medical device. In another example, the entire medical device may be inserted inside the patient's body, where the distal end of the medical device may extend further inside the patient's body than the proximal end of the medical device.

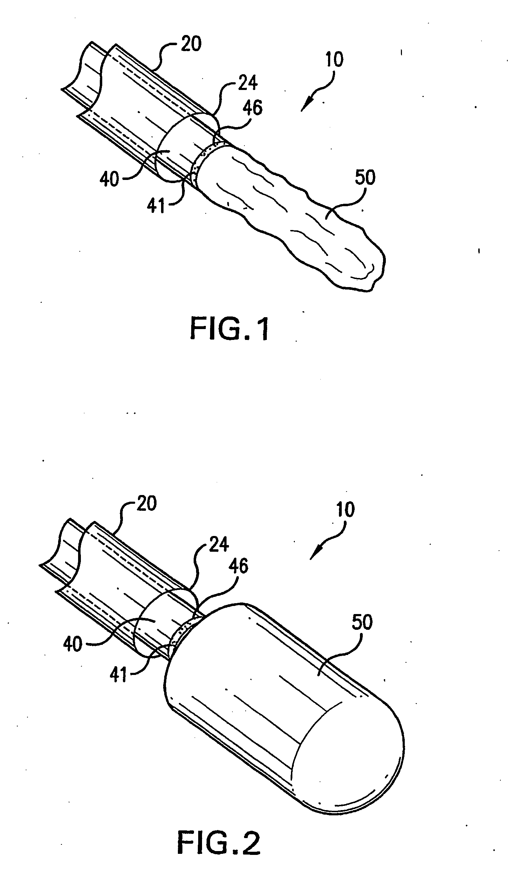

Referring now to the Figures, in which like part numbers depict like elements throughout the Figures, Figure 1 is a perspective view of a system 10. The system 10 shown in Figure 1 is configured to allow an operator to provide, alter or maintain a dimension of a cavity in a treatment area. The system 10 is further configured to be used in a kyphoplasty procedure to restore height to a vertebra suffering from a vertical compression fracture condition.

The system 10 comprises a hollow member 20 comprising a proximal end (not shown) and a distal end 24. The hollow member 20 may be fabricated from a material selected to facilitate advancement and rotation of an elongate member 40 movably disposed within the hollow member 20. The hollow member 20 may comprise, for example, a surgical cannula of suitable inner diameter to perform various surgical procedures. The hollow member 20 can be constructed, for example, using standard flexible, medical grade plastic materials, such as vinyl, nylon, polyethylenes, ionomer, polyurethane, and polyethylene tetraphthalate (PET). At least some portion of the hollow member 20 can also comprise more rigid materials to impart greater stiffness and thereby aid in its manipulation and torque transmission capabilities. More rigid materials that can be used for this purpose comprise stainless steel, nickel-titanium alloys (such as Nitinol), and other metal alloys.

The system 10 shown in Figure 1 further comprises the elongate member 40 configured to be maneuverable along a path established by the hollow member 20. The elongate member 40 may be made from a resilient inert material providing torsion transmission capabilities (e.g., stainless steel, a nickel-titanium alloy such as Nitinol, and other suitable metal alloys). In other versions, the elongate member 40 may be fashioned from a variety of suitable materials, comprising a carbon fiber, a glass, or a flexible material, such as a plastic or rubber. In one version comprising a flexible elongate member 40, the elongate member 40 may be, for example, fashioned from twisted wire filaments, such stainless steel, nickel-titanium alloys (such as Nitinol), and suitable other metal alloys.

The elongate member 40 shown is hollow, allowing movement of a flowable material within a bore therethrough along its axis. A flowable material may comprise, for example, a liquid material, a gaseous material, a slurry, a sludge, a plasma, a paste, a flowable solid (such as powdered, pulverized, granulated, pelletized, or encapsulated material), or any other suitable material that may flow naturally or be made to flow from one place to another. The elongate member 40 shown comprises a fitting 41 at its distal end 46. The elongate member 40 may comprise a handle (not shown) at its proximal end (not shown) to aid in gripping and maneuvering the elongate member 40. For example, in one version, such a handle can be made of a foam material secured about the proximal end elongate member 40.

The system 10 shown in Figure 1 further comprises an expansible body 50. The expansible body 50 may be fashioned from a variety of suitable body materials that are substantially insoluble to a biocompatible filler material. The biocompatible filler material may comprise, for example, a bone cement material, such as polymethylmethacrylate (PMMA), PMMA mixed with barium and/or hydroxyapatite, calcium phosphate, calcium sulphate, allograft, or a self-setting polyurethane.

Suitable body materials for the expansible body 50 may be strong and flexible. For example, the expansible body 50 may comprise a body material comprising one or more plastics, such as flexible PVC (polyvinyl chloride), cross-linked polyethylene, PET, nylon, or Mylar. In other versions, the body material may comprise, for example, one of the following monomer resistant materials: polyurethanes, polyamides, polyesters, fluoropolymers, polyethylenes, polyproplenes, polyvinyl chlorides, polyetheretherketones, polyetherimides, and polyethersulfones.

In some versions, the body material may comprise, for example, a non-porous material. In other versions, the body material may be porous and configured to allow communication of a flowable material between an interior volume of the expansible body 50 and a treatment area.

In some versions, the expansible body 50 may be coated, impregnated, or embedded with a material to aid in its use. A coating material may be applied, for example, at a factory configured to produce at least the expansible body 50 or by a user of the device prior to use in a patient. For example, the expansible body 50 may be coated or impregnated with silicone or Teflon for lubrication, for abrasion resistance, or may be coated or impregnated with a therapeutic material, such as an anticoagulatory drug. In other versions, the expansible body 50 may comprise, for example, a body material that is rupture-resistant, resistant to an exothermic reaction caused by curing of a biocompatible filler material, or a material that substantially resists adhesion to a biocompatible filler material.

In yet other versions, the expansible body 50 may comprise a body material that is configured to substantially adhere to a biocompatible filler material. For example, in one such version, the expansible body 50 may be permanently deployed in an intervertebral disc, and a biocompatible filler material may be used to secure the expansible body 50 to one or more tissues in the treatment area. For example, a biocompatible filler material may be configured to substantially adhere to both the expansible body 50 and to an annulus of a patient's intervertebral disc, thereby securing the expansible body 50 to the patient's annulus.

The expansible body 50 may be fashioned by extruding material into a tube shape, and then forming the tube into a balloon through blow molding. For example, raw materials in granulated form may be heated to melt and liquefy them, and a rotating screw may mix the materials into a homogeneous blend. The liquid material may then be pumped through an extrusion device to extrude the expansible body 50. The extruded material may then be pulled by a mechanical puller through a cooling bath, freezing it in a solidified form. A mechanical cutter may be used to chop the tubing to its specified length.

The expansible body 50 may then be formed from the extruded material through blow molding using a variety of different sizes of glassforms, each configured to provide an expansible body 50 of a suitable diameter. One end of the expansible body 50 may then be welded shut. The finished expansible body 50 may be inspected for quality control, sterilized, and collapsed using a vacuum pump. The expansible body 50 may then be disposed at the distal end 46 of the elongate member 40, for example by gluing or heat bonding. In the version shown in Figures 1 and 2, the expansible body 50 comprises a single aperture that is coupled to a fitting 41 at the distal end 46 of the elongate member 40.

The expansible body 50 is shown in Figure 1 in a collapsed state. The expansible body 50 is configured to be maneuverable along a path established by the hollow member 20 while in acollapsed state, as shown in Figure 1. The expansible body 50 shown in Figure 1 is configured to be inserted adjacent a tissue in a treatment area through the hollow member 20.

In one version of the present invention, the expansible body 50 may be configured to be deployed adjacent cancellous bone tissue within a vertebral body through a percutaneous path established by the hollow member 20. For example, the expansible body 50 may be deployed within a cavity formed by another device in a vertebral body, where the expansible body 50 is configured to maintain a dimension of the cavity at least while a biocompatible filler material is disposed therein.

Referring still to Figure 1, the expansible body 50 may be expanded by movement of a flowable material through the hollow elongate member 40 and the through the aperture into the interior of the expansible body 50. Conversely, the expansible body 50 may be contracted by movement of a flowable material out of the expansible body 50 through the aperture and the bore through the hollow elongate member 40.

The expansible body 50 shown in Figures 1 and 2 comprises a round cross section. In other versions, the expansible body 50 can, in cross section, be polygonal, rectilinear, ovoid, asymmetrical, or any other suitable configuration. While in the collapsed state shown in Figure 1, the expansible body 50 comprises a collapsed dimension. As shown in Figure 1, while the expansible body 50 is in the collapsed state, it fits within the inside bore dimension of the hollow member 20, thereby providing a clearance. Such a clearance may allow a user of the system 10 to maneuver the elongate member 40, and thereby the expansible body 50, within and along the axis of the hollow member 20. The elongate member 40 and the expansible body 50 shown in Figure 1 are configured to be rotated with respect to the hollow member 20 if the user so desires.

Referring now to Figure 2, a perspective view of the system 10 shown in Figure 1, wherein the expansible body 50 thereof is shown in an expanded state. While in the fully expanded state shown in Figure 2, the expansible body 50 is configured to have a predetermined expanded dimension and shape. In one version, a user of the system 10 may select the expansible body 50 from a plurality of selectable expansible bodies 50, each configured to have a unique predetermined expanded dimension or shape.

A user in such a version may select the expansible body 50 based, at least in part, on a volume of a flowable material used to provide a cavity in a treatment area using an inflatable balloon tamp, or on a configuration of tissues in the treatment area. For example, the expansible body 50 may be shorter in length (as measured along the axis of the elongate member 40) but greater than or equal to a diameter dimension of a cylindrical inflatable balloon tamp used to provide a cavity within and restore height to a vertebral body treatment area prior to insertion of the expansible body 50 to permit the expansible body 50 to maintain a dimension of a cavity in the treatment area while permitting a biocompatible filler material to fill part of the cavity. The expansible body 50 is configured to be expanded from the collapsed dimension shown in Figure 1 to the predetermined expanded dimension shown in Figure 2 when extended to a point beyond the distal end 24 of the hollow member 20.

The expansible body 50 shown in Figures 1 and 2 is further configured to be contracted from the predetermined expanded dimension shown in Figure 2 to the collapsed dimension shown in Figure 1 prior to or when brought within the distal end 24 of the hollow member 20 from a point beyond the distal end 24 of the hollow member 20. For example, in one version, the expansible body 50 may be contracted prior to removing it from a treatment area through the hollow member 20.

As shown in Figure 2, when expanded, the expansible body 50 is constrained from further expansion by the substantially non-compliant body material from which it is fabricated. In a different version, the expansible body 50 may be fashioned from a compliant material, such as latex. The expansible body 50, as shown in Figure 2, is constrained to a substantially circular cross section. In other versions, the expansible body 50 may be constrained to a different cross-sectional configuration or dimension. For example, in one version, the expansible body 50 may be constrained to a substantially rectangular or triangular cross section. The expansible body 50, in the version shown in Figure 2, is configured to have a predetermined expanded dimension, D, as measured perpendicular to the axis of the elongate member 40. In the version shown in Figure 2, the fully expanded dimension, D, is larger than the inside diameter of the interior bore of the hollow member 20.

In one version, the expansible body 50 may be substantially cylindrical in shape, but may comprise a passage therethrough. A flowable material may pass through such a passage. For example, a biocompatible filler material, such as PMMA may pass through such an expansible body 50 while the expansible body maintains a dimension of a cavity in a treatment area. Such a passage may be configured to aid the efficiency or accurateness of a process used to at least partially fill a cavity in a treatment area with a biocompatible filler material.

In another version at least a portion of the expansible body 50 may comprise at least one surface that is configured to contact and shear (curette) or abrade a tissue when the expansible body 50 is expanded. In one such version, while the expansible body 50 is expanded, the elongate member 40 may be rotated in the hollow member 20, thereby rotating the expansible body 50. In yet another version, at least a portion of the elongate member 40 may comprise a surface configured to directly contact and shear or abrade a tissue.

In a version comprising an edge configured to contact and shear or abrade a tissue, the expansible body 50 may be configured to cut or dislodge adjacent tissue mass in the treatment area when rotated, providing or enlarging a cavity in the tissue. In another version, the proximal end of at least one of the hollow member 20 and the elongate member 40 may carry a fitting (not shown) that, in use, may be coupled to an electric motor (not shown). The motor may thus rotate one or both of the elongate member 40 and the hollow member 20, thereby rotating the expansible body 50.

In another version of the present invention, at least a portion of the expansible body 50, the elongate member 40, or the hollow member 20 may comprise one or more radiological markers. A radiological maker may permit radiologic visualization of at least one of the elongate member 40, the expansible body 50, and the hollow member 20 within a treatment area. The markers may be fashioned from a radiopaque material, such as platinum, gold, calcium, tantalum, and other heavy metals. In other versions, other forms of markers can be used to allow a user to visualize the location, size, and shape of at least the expansible body 50 within the treatment area. For example, the expansible body 50 may be expanded with a radiopaque gas or liquid.

A system, such as the system 10 described with respect to Figures 1 and 2, can comprise an interior lumen. The lumen may be coupled to an external source of fluid and an external vacuum source. In one such version, a rinsing liquid, e.g., sterile saline, can be introduced from the source through the lumen into the tissue region before, during or after the system 10 provides, alters, or maintains a cavity in a tissue mass. A rinsing liquid may reduce friction and conduct heat away from the tissue. The rinsing liquid can be introduced continuously or intermittently while the tissue mass is being compacted, removed, or cut. The rinsing liquid can also carry an anticoagulant or other anti-clotting agent. In one such version, the lumen may be coupled to the vacuum source, and liquids and debris can be aspirated from the tissue region through the lumen.

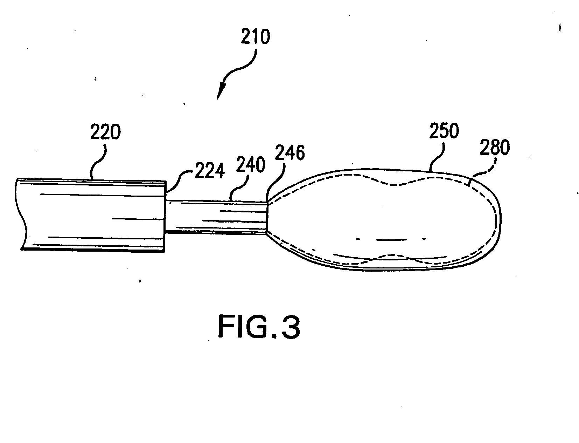

In yet another version, the expansible body 50 may comprise a first, outer expansible body, and may surround a second, inner expansible body (see Figure 3), such as the KyphX® Xpander® inflatable balloon tamp (IBT). In one such version, the expansible body 50 may comprise a sheath, and may be at least partially coupled to an inner expansible body. For example, the outer expansible body 50 may be fabricated from a substantially non-compliant and rupture-resistant body material (e.g., nylon or Mylar), that is configured to be substantially insoluble to a biocompatible filler material or resistant to a monomer therein, while the inner expansible body is compliant and is fashioned from a material that is soluble to or otherwise negatively affected by a biocompatible filler material or a curing process thereof (e.g., an exothermic curing process of a PMMA bone cement). For example, in some applications, an inner expansible body may be fashioned from a compliant polyurethane-based material.

An expansible body 50 according to such an version may constrain expansion of an inner expansible body. For example, the expansible body 50 may comprise at least one fully expanded dimension that is lesser than a corresponding dimension that a compliant inner expansible body would be able to achieve if it were not sheathed in the expansible body 50. In another version, the expansible body 50 may be configured to loosely and flexibly wrap an inner expansible body. In such a version, the expansible body 50 may be configured to expand and contract in accordance with the inner expansible body. The sheath-like expansible body 50 may also prevent dislodged tissue mass from puncturing the inner expansible body.

Referring now to Figure 3, an elevation view of a system 210 is shown. As shown in Figure 3, the system 210 comprises a hollow member 220. The hollow member 220 may comprise, for example, a surgical cannula similar to the hollow member 20 described with respect to Figures 1 and 2. The system 210 further comprises an elongate member 240. The elongate member 240 may comprise, for example an elongate member similar to the elongate member 40 described with respect to Figures 1 and 2.

The system 210 further comprises a first outer expansible body 250 disposed at a distal end 246 of the elongate member 240. The first expansible body 250 is configured to be maneuverable along a path established by the hollow member 220. The first expansible body 250 shown is configured to have a predetermined expanded dimension. The first expansible body 250 further comprises a body material substantially insoluble and resistant to a biocompatible filler material, such as calcium sulphate.

The system 210 also comprises a second inner expansible body 280 disposed within the first expansible body 250. The second expansible body 280 may comprise, for example, a KyphX® Elevate^{™} IBT device. The second expansible body 280 and the surrounding first expansible body 250 are shown extended beyond a distal end 224 of the hollow member 220. The second expansible body 280, as shown in Figure 3, is expanded with a material (such as a radiopaque liquid as described above) through an aperture (not shown) facing the distal end 246 of the elongate member 240.

In the version shown, the second expansible body 280 is configured to be expanded, thereby expanding the first expansible body 250. The first expansible body 250 is configured to constrain expansion of the second expansible body 280 as the second expansible body 280 expands. In a different version, the first expansible body 250 may be configured to provide substantially no restraint to the expansion of the second expansible body 280. In such a version, the first expansible body 250 may act as a barrier between the second expansible body 280 and a biocompatible filler material.

Referring still to Figure 3, the first and second expansible bodies 250, 280 are configured to be expanded once inserted through the hollow member 220 to a point beyond the distal end 224 of the hollow member 220 as shown in Figure 3. A biocompatible filler material may be inserted into a treatment area while the first and second expansible bodies 250, 280 maintain an expanded dimension of the treatment area. In another version, at least a portion of the first expansible body 250 may be coupled to the second expansible body 280. In yet another version, the second expansible body 280 may be contracted while the first expansible body 250 remains expanded.

In the version shown in Figure 3, the first expansible body 250 is further configured to collapse as the second expansible body 280 contracts. The contracted second expansible body 280 and the collapsed first expansible body 250 may then be withdrawn into the distal end 224 of the hollow member 220. The elongate member 240 may then be withdrawn along a path established by the hollow member 220, thereby removing the first expansible body 250 and the second expansible body 280 disposed within from the treatment area.

The device 210 shown in Figure 3 may comprise a controller (not shown). At least some portion of the first expansible body 250, the second expansible body 280, or the elongate member 240 may be in communication with one or more suitable types of controller, such as a slide controller, a pistol grip controller, a ratcheting controller, a threaded controller, or any other suitable type of controller that can be configured to permit an operator of the system 210 to control at least one of the extent to which the first or second expansible bodies 250, 280 extend beyond the distal end 224 of the hollow member 220, or the extent to which the first or second expansible bodies 250, 280 are expanded or contracted.

For example, a controller may be configured to allow an operator of the device 210 to adjust the volume of liquid in the second expansible body 280. By adjusting the volume of liquid in the second expansible body 280, an operator of the system 210 may expand or contract the first expansible body 250. In one version, a controller can also comprise indicia by which the physician can visually estimate the extent to which a controlled element has been adjusted.

In the version shown in Figure 3, the device 210 may be used to provide, adjust, or maintain a cavity in an interior body region. A user of the device 210 may be able to use the controller to adjust the size and shape of the second expansible body 280, and thereby the first expansible body 250 within the cavity. For example, by expanding the second expansible body 280, thereby expanding the surrounding first expansible body 250 within the treatment area using the controller, the user may be able to provide a force to the surrounding tissues to provide a cavity of desired shape and dimension. In a different version, a user may be able to adjust the size or shape of the first expansible body 250 substantially independently of the second expansible body 280.

In one version, the first expansible body 250 may be fashioned from a material that is stronger but less compliant than the second expansible body 280. In such an version, the first expansible body 250 may act to prevent a rupture of the second expansible body 280, thereby allowing a user of the device 210 to inflate the second expansible body 280 with a greater interior pressure than would be possible without using the first expansible body 250. Similarly, in another version, the first expansible body 250 may prevent sharp, jagged, or pointed tissue materials, such as bone fragments, from rupturing a more susceptible and compliant second expansible body 280.

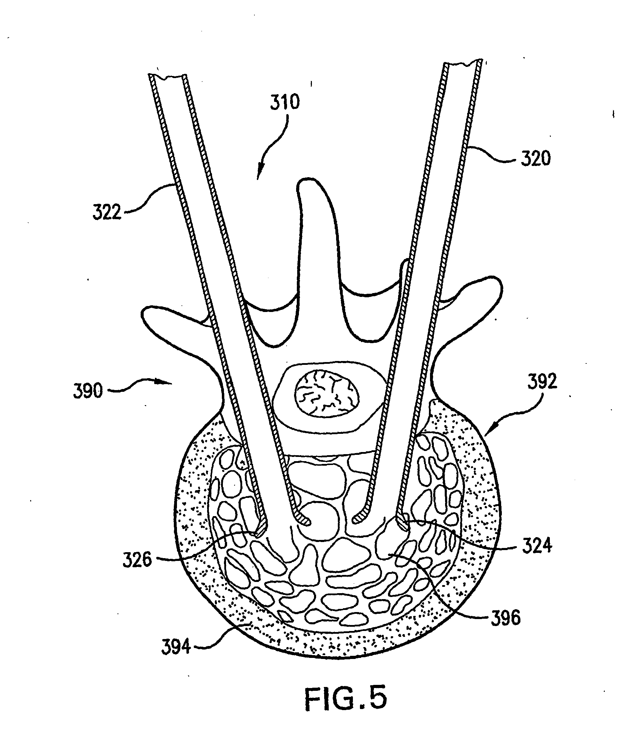

A biocompatible filler material, such as a bone cement, may be used to fill at least a portion of a cavity formed by a device according to an embodiment of the present invention within a treatment area. The first expansible body 250 may be fashioned from a body material substantially insoluble to or resistant to an exothermic curing process of a biocompatible filler material (e.g., nylon or Mylar). Use of such a body material may allow at least the first expansible body 250 to remain expanded in a treatment area while the biocompatible filler material is inserted into the treatment area without releasing the contents of the first expansible body 250. Such an embodiment may be useful in situations where the system 210 is used to restore height to a vertebral body (see Figures 5-7). The biocompatible filler material may be inserted, either via the hollow member 220, or via a separate hollow member (such as a contralateral hollow member).

In one embodiment, upon conclusion of use within a treatment area, the second expansible body 280 may be contracted, thereby collapsing the first expansible body 250. The first expansible body 250 and the second expansible body 280 may then be removed from the interior body region through the hollow member 220. In another embodiment, the second expansible body 280 may be contracted and removed while the first expansible body 250 at least temporarily remains at least partially expanded within the treatment area.

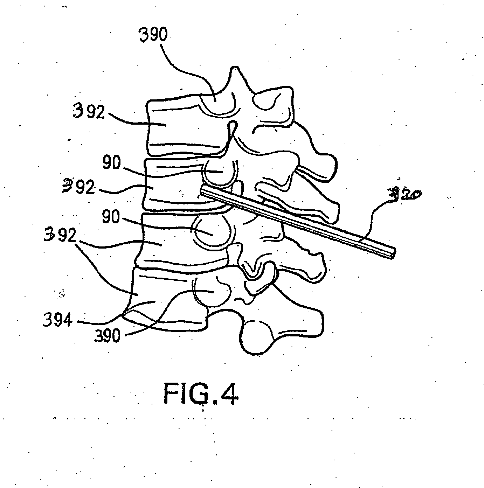

Referring now to Figure 4, an elevation (lateral) view of several human vertebrae 390 is shown, with a hollow member 320 establishing a percutaneous path along its axis to a vertebral body 392 of one of the several vertebrae. The vertebral body 392 extends on the anterior (i.e., front or chest) side of the vertebra 390. The vertebral body 392 comprises an exterior formed from compact cortical bone 394. The cortical bone 394 encloses an interior volume of reticulated cancellous, or spongy, bone 396 (also called medullary bone or trabecular bone - shown in Figures 5-7).

The vertebral body 392 is in the shape of an oval disc. As Figures 4-7 show, access to the interior volume of the vertebral body 392 can be achieved, e.g., by drilling an access portal through a rear side of the vertebral body 392, (a posterolateral approach). The portal for the postero-lateral approach enters at a posterior side of the vertebral body 392 and extends anteriorly into the vertebral body 392. The portal can be provided either with a closed, minimally invasive procedure or with an open procedure.

Alternatively, access into the interior volume can be accomplished by drilling an access portal through one or both pedicles of the vertebra 390. This is called a transpedicular approach, and is illustrated in Figures 5-7. It is the physician who ultimately decides which access site is indicated. Access into the interior of the vertebral body may also be accomplished using an extrapedicular approach alongside a pedicle of the vertebra 390, or by approaching the vertebra 390 from its anterior side. It is the physician who ultimately decides which access site is indicated.

A tool according to the present invention may be configured to be deployed within a treatment area within or adjacent at least one layer of tissue by movement within and along a path formed by the axis of the first hollow member 320. For example, as shown in Figure 4, the first hollow member 320 may provide a tool, such as at least part of the systems 10 or 210 described above, with access to the cancellous bone within the vertebral body 392 of a vertebra 390 to provide a cavity therewithin. Such a cavity may be provided during a procedure for restoring some of the height of a vertebral body lost due to a vertical compression fracture or other pathology or trauma, prior to insertion of a biocompatible filler material, such as a bone cement, into the vertebral body 392.

It should be appreciated, however, that systems and methods according to the present invention are not limited in application to human vertebrae, and may be used to provide cavities within or curette other parts of a living or non-living organism. For example, the systems 10 or 210 can be deployed in other embodiments in other bone types and within or adjacent other tissue types, such as in a vertebral disc, a knee joint, etc.

Referring now to Figure 5, a plan (coronal) view of a vertebra 390 being accessed by a system 310 according to one embodiment of the present invention is shown. The vertebra 390 is shown, with portions removed to reveal cancellous bone 396 within a vertebral body 392. As shown in Figure 5, the system 310 is shown being used to perform a kyphoplasty procedure on the vertebra 390. In other embodiments, the system 310 may be used to perform another procedure in a different type of bone or another body tissue.

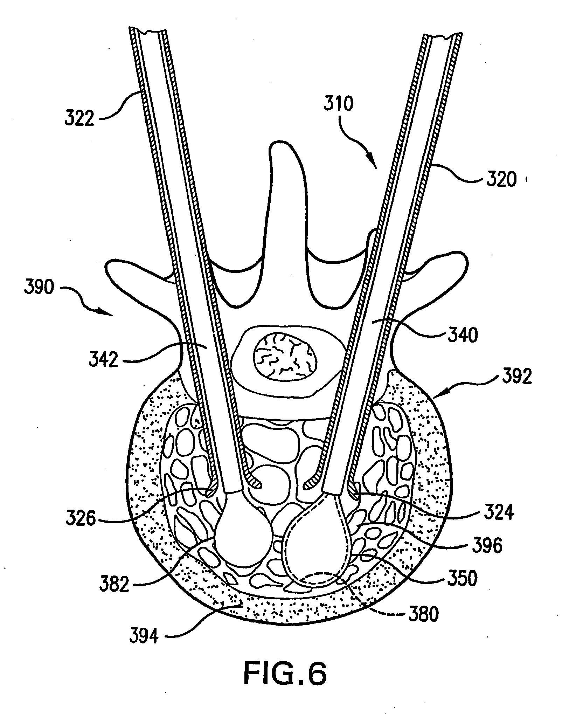

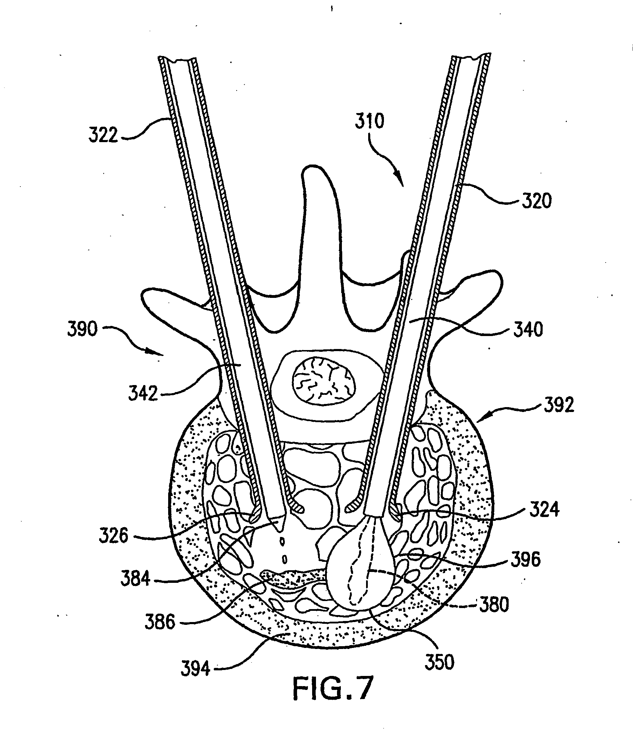

As shown in Figure 5, a first hollow member 320 and a second hollow member 322 have been inserted into the vertebral body 392 of the vertebra 390. The system 310 is similar to the system 210 described above with respect to Figure 3. The system 310 differs from the system 210 in that, while comprising similar elements (a first elongate member 340, a second elongate member 342, a first expansible body 350, a second expansible body 380, and a third expansible body 382 - see Figures 6 and 7), the first expansible body 350 and the second expansible body 380 disposed therewithin are independently expansible and adjustable.

The first and second hollow members 320 and 322 are shown in Figures 5-7 with portions removed to reveal other elements of the system 310 (the elongate members 340, 342; the first expansible body 350, and the second and third expansible bodies 380, 382 - see Figures 6 and 7) that are configured to be maneuverable along paths established by the hollow members 320, 322. These elements are configured to move within and along the axis of the hollow members 320, 322 to gain access to a treatment area beyond a first distal end 324 of the first hollow member 320 and a second distal end 326 of the second hollow member 322.

Referring now to Figure 6, a plan (coronal) view of the vertebra 390 is shown. The system 310 comprises the first elongate member 340. The first elongate member 340 is shown within the first hollow member 320. The system 310 further comprises the second elongate member 342. The second elongate member 342 is shown within the second hollow member 322. In use, the elongate members 340, 342 are substantially carried for sliding and rotation within the hollow members 320, 322, respectively. For example, a user of the system 310 may maneuver the first elongate member 340 along the path established by the axis of the first hollow member 320 to deploy the first expansible body 350 and the second expansible body 380 disposed therewithin in a treatment site.

The first expansible body 350 is disposed at the distal end 346 of the first elongate member 340. The first expansible body 350 may comprise, for example, the first expansible body 50 described above with respect to Figures 1 and 2. Disposed within the first expansible body 350 is the second expansible body 380. In another embodiment, the second expansible body 380 may have been used without the first expansible body 350 to provide a cavity within the vertebral body 392 and then removed. The first expansible body 350 (without the second expansible body 380) may have then been inserted into the cavity to maintain an increased dimension provided by the second expansible body 380.

When deployed in the treatment site, the user can expand the second expansible body 380 shown in Figure 6, thereby at least partly expanding the first expansible body 350 adjacent cancellous bone tissue 396 within the vertebral body 392. The user may also be able to rotate the first elongate member 340 within the first hollow member 320 and thereby the second expansible body 380 and the first expansible body 350 to adjust at least one of their orientation and travel path.

The system 310 further comprises the third expansible body 382 disposed at the distal end of the second elongate member 342. The third expansible body 382 is in communication with a channel extending through the second elongate member 342. The third expansible body 382 is configured to be expanded by a radiopaque fluid material passing through the channel in the second elongate member 342 into the third expansible body 382 when the third expansible body 382 is extended beyond the distal end 326 of the second hollow member 322.

In other embodiments, at least one of the first expansible body 350, the second expansible body 380, the third expansible body 382, the first elongate member 340, the second elongate member 342, the first hollow member 320, or the second hollow member 322 can carry one or more radiological markers, as previously described. The markers may allow radiologic visualization of various elements of the system, and their positions or dimensions relative to each other or the vertebra 390 while in use within a treatment area.

In the embodiment shown, the second expansible body 380 and the first expansible body 350 are in communication with separate channels extending through the first elongate member 340 such that the first expansible body 350 may be expanded independently of the second expansible body 380. In another embodiment, the first expansible body 350 may comprise a flexible sheath configured to prevent contact between the second expansible body 380 and a biocompatible filler material, where the first expansible body 350 is unable to expand or contract independently of the second expansible body 380.

As shown in Figure 6, the first expansible body 350 has been deployed within the cancellous bone 396 in the vertebral body 392 to provide a first cavity therewithin. For example, the vertebral body 392 of the vertebra 390 may have been partially crushed due to an osteoporotic condition of cancellous bone 396 therewithin. A user of the system 310 may wish to use it to restore height to the vertebral body 392 lost when the fracture occurred.

In the embodiment shown in Figure 6, the second expansible body 380, and the first expansible body 350 have been inserted into the vertebral body 392 through the first hollow member 320 in an collapsed state. Once beyond the distal end 324 of the first hollow member 320, a radiopaque liquid material has been inserted into the second expansible body 380 disposed within the first expansible body 350, thereby expanding both the first and second expansible bodies 350, 380, and providing a first cavity within the cancellous bone 396. As shown in Figure 6, a gaseous material has been inserted into the space between the second expansible body 380 and the first expansible body 350.

Similarly, the third expansible body 382 is shown expanded within the cancellous bone 396 of the treatment area. The third expansible body 382 has provided a second cavity within the cancellous bone 396. In another embodiment, the third expansible body 382 may also be disposed within an expansible body comprising a body material substantially insoluble or resistant to a biocompatible filler material.

As shown in Figure 6, there is some cancellous bone tissue 396 between the first and second cavities provided by the first expansible body 350 and the third expansible body 382, respectively. However, due to the osteoporotic condition of the vertebra 390, the cancellous bone 396 has many passages therethrough, and a flowable material, such as a biocompatible filler material, inserted into one of the cavities may pass through the porous cancellous bone 396 to the other cavity.

Referring now to Figure 7, a plan (coronal) view of the vertebra and system shown in Figures 5 and 6, wherein a biocompatible filler material 386 is shown being inserted into one of the cavities. As shown in Figure 7, the first expansible body 350 is shown in a fully expanded state, while the second expansible body 380 disposed therewithin has been contracted. In another embodiment, the second expansible body 380 may remain at least partially expanded.

As seen in Figure 7, the third expansible body 382 (not shown) has been contracted and removed along with the second elongate member 342 from the treatment area through the second hollow member 322. In its stead, a filler tube 384 has been inserted into the second hollow member 322. The filler tube 384 is configured to insert a liquid biocompatible filler material 386 into the second cavity provided by the third expansible body 382. The biocompatible filler material 386 may comprise a bone cement, such as polymethylmethacrylate (PMMA).

The first expansible body 350 is configured to maintain a dimension of the first cavity in the cancellous bone 396 while the biocompatible filler material 386 is inserted into the second cavity by the filler tube 384 through the second hollow member 322. By maintaining the dimension of the first cavity while the biocompatible filler material 386 is inserted into the second cavity, the system 310 may permit a user to restore the vertebra 390 to a shape more analogous to a pre-vertical compression fracture condition than inserting the biocompatible filler material 386 without maintaining the dimension of the first cavity with the first expansible body 350.

For example, once the third expansible body 382 has been contracted and removed from the cancellous bone 396 in the vertebral body 392, the force of gravity, the mass of the patient, the configuration or condition of the patient's bones or other body structures, or another factor may provide a force that tends to decrease a distance between cortical bone 394 endplates located above and below the cancellous bone 396 of the vertebral body 392. The presence of the first expansible body 350 may prevent, at least in part, such a force from decreasing the distance between the endplates.

As shown in Figure 7, the biocompatible filler material 386 is in contact with the first expansible body 350. The first expansible body 350 comprises a body material (such as Mylar or nylon) that is substantially insoluble to the biocompatible filler material 386, and substantially resistant to an exothermic reaction caused by a curing process of the biocompatible filler material 386. Accordingly, contact between the biocompatible filler material 386 and the first expansible body 350 will not dissolve or weaken the first expansible body 350. The gaseous material within the first expansible body 350 may thus be prevented from escaping into the surrounding tissue in the treatment area.

In one embodiment, a suction tube may also be deployed along a path established by either the first hollow member 320 or the second hollow member 322 to remove cancellous bone 396 dislodged when the first expansible body 350 or the third expansible body 382 provided the first and second cavities, respectively. In yet another embodiment, the system 310 may comprise an interior lumen to serve as a suction tube as well as to convey a rinsing liquid into the treatment area as cavities are being formed or filled therein: The suction tube (or a lumen) may introduce a rinsing fluid (with an anticoagulant, if desired) and may remove loosened cancellous bone 396. Alternatively, one or both of the hollow members 320, 322 may comprise a first interior lumen that serves as a suction tube, or a second interior lumen that serves to flush the treatment area. In one embodiment, by periodically inflating or deflating the first balloon 380 and the first expansible body 350, a user of the system 310 may provide a cavity within the treatment area having a dimension desired by the user.

Once the second cavity has been filled with the biocompatible filler material 386, and the biocompatible filler material 386 has set or hardened, the first expansible body 350 may be contracted. The first elongate member 340 may then be removed from the first hollow member 320, thereby removing the first expansible body 350 and the second expansible body 380 disposed therein from the treatment area. In one embodiment, the first cavity provided by the first expansible body 350 and the second expansible body 380 may then be at least partially filled with the biocompatible filler material 386, or another suitable material.

Another suitable tool can be deployed through the first or second hollow members 320, 322 into the treatment area. For example, in one embodiment, another tool may, for example, perform a diagnostic or therapeutic procedure (such as providing a therapeutic material to the tissues in the treatment area). In one embodiment, at least a portion of the first expansible body 350 may be coated with a therapeutic material. When inserted into the treatment area, the expansible body may thus provide a therapeutic effect to a tissue therein by contacting the tissue and depositing some of the therapeutic material thereon. For example, an allograft material, a synthetic bone substitute, a medication, or a biocompatible flowable material that may set to a hardened condition may be provided to some portion of a treatment area by an expansible body according to one embodiment of the present invention.

In one embodiment, the system 310 may also be used to apply radiation therapy or chemotherapy. Further details of the injection of such materials into a treatment area for therapeutic purposes may be found in U.S. Pat. Nos. 4,969,888 and 5,108,404, and in copending U.S. patent application Publication No. 2003/0229372.

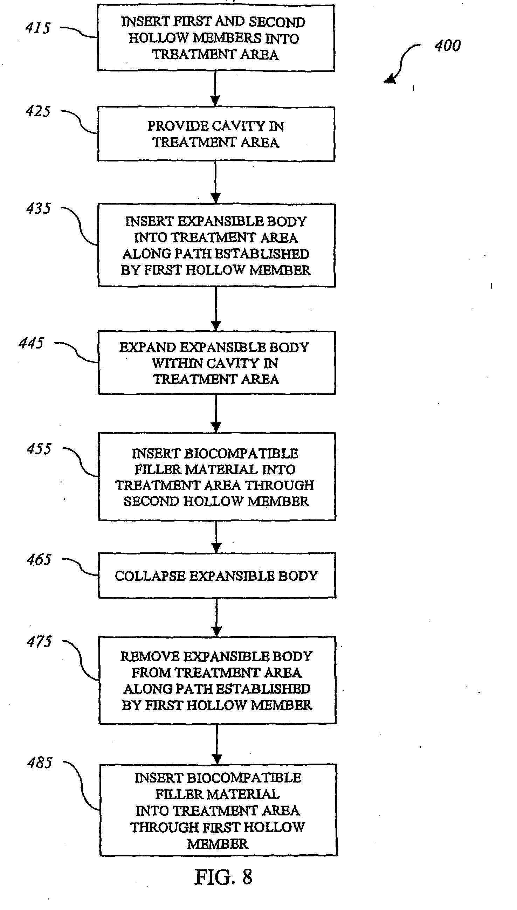

Referring now to Figure 8, a flow chart of a treatment method 400 is shown. The illustrative method comprises percutaneously inserting a first hollow member and a second hollow member into a treatment area within a vertebral body of a vertebra comprising a vertical compression fracture condition, as shown in box 415. The first and second hollow members may each comprise, for example, a surgical cannula similar to the hollow members 320, 322 described above.

The method 400 further comprises providing a cavity within the treatment area, as shown in box 425. For example, in one embodiment, a surgeon may insert KyphX® Xpander® inflatable balloon tamps (IBT) into both the first and second hollow members, and expand these devices to restore height lost when the vetebra fractured, and to provide a cavity within the vertebral body during a kyphoplasty procedure.

The method 400 further comprises inserting an expansible body comprising a body material substantially insoluble and resistant to a biocompatible filler material into the treatment area along a path established by the first hollow member, as shown in box 435. The expansible body may be positioned within the cavity provided by another device. The expansible body may comprise, for example, the expansible body 50 described above with respect to Figures 1 and 2, or the first expansible body 250 described with respect to Figure 3. The body material of the expansible body may comprise, for example, Mylar, nylon, or PET.

In one treatment method, an expansible body comprising a body material substantially insoluble and resistant to a biocompatible filler material may be inserted into the treatment area after an expansible body fashioned from a material that is compliant (e.g., a polyurethane-based material) and degrades or weakens when exposed to the biocompatible filler material has been inserted into the treatment area, expanded to provide a cavity therewithin, contracted and removed. The expansible body comprising the body material that is substantially insoluble and resistant to the biocompatible filler material may then be inserted into the cavity to maintain a dimension of the cavity while the cavity is filled with the biocompatible filler material.

In another treatment method, an expansible body comprising a body material that is substantially insoluble and resistant to a biocompatible filler material may comprise a first expansible body, wherein a second expansible body is disposed therewithin, similar to that described with respect to Figure 3. The inner second expansible body may be expanded, either independently or in conjunction with the first expansible body. The first expansible body may thereby be expanded.

The method 400 further comprises expanding the expansible body within the cavity provided in the treatment area, as shown in box 445. The expansible body may be expanded once extended beyond a distal end of the hollow member and inside the treatment area in the vertebral body. The expansible body comprising a body material that is substantially insoluble and resistant to a biocompatible filler material may be expanded within a cavity previously provided by another device (such as a curette or a compliant expansible body comprising a body material susceptible to weakening or dissolving when contacted by the biocompatible filler material) within the treatment area.

The expansible body may be configured to have a predetermined expanded dimension. In such an embodiment, expanding the expansible body within the treatment area may comprise expanding the expansible body to the predetermined expanded dimension. For example, a user of the method 400 may select an expansible body from a plurality of expansible bodies, each comprising a unique expanded dimension. Selection of one of the plurality of expansible bodies may be based, at least in part, on a size or shape of another tool previously inserted into the treatment area, or a size or shape of a cavity previously provided within the treatment area by another tool, such as a compliant expansible body that is soluble to a biocompatible filler material.

The method 400 further comprises inserting a biocompatible filler material into the treatment area through the second hollow member while the expansible body is expanded, as shown in box 455. The biocompatible filler material may comprise, for example, a bone cement material, such as polymethylmethacrylate (PMMA), PMMA mixed with barium and/or hydroxyapatite, calcium phosphate, calcium sulphate, allograft, or a self-setting polyurethane. Because the expansible body comprises a body material that is substantially insoluble and resistant to the biocompatible filler material, curing reactions thereof, and monomers therein, the expansible body may be expanded or otherwise filled with a flowable material (such as a liquid material or a gaseous material) without weakening or dissolving when contacted by the biocompatible filler material. Materials within the expansible body may thus be kept from contacting a tissue adjacent the expansible body within the treatment area.

For example, in one treatment method to obtain the maximum possible height restoration of a vertebral body suffering from a vertical compression fracture condition, a user may wish to insert an expansible body into a cavity provided within one side of a vertebral body while a cavity within an opposing side of the vertebral body is filled with a bone cement biocompatible filler material. The expansible body may be expanded by inflation with a radiopaque liquid material, for example. While expanded, the expansible body may be configured to maintain a maximum dimension of the treatment area while the bone cement biocompatible filler material is inserted into the vertebral body prior to hardening.

However, due either to provision of cavities or to existing channels within the vertebral body, a biocompatible filler material, while liquid, may travel through passages or apertures within the vertebral body to come into contact with body material of an expansible body. The substantial insolubility and resistance of the body material to the biocompatible filler material may prevent the expansible body from weakening or dissolving, thereby maintaining the size and shape of the expansible body, and isolating tissue in the treatment area from a flowable material contained within the expansible body.

Referring still to Figure 8, the method 400 further comprises collapsing the expansible body, as shown in box 465. For example, if the expansible body has been filled with a gaseous material, the expansible body may be contracted by evacuation of the gaseous material by a vacuum in communication therewith. In one treatment method, the expansible body may be collapsed once a user has determined that a biocompatible filler material inserted into the treatment area has achieved its desired purpose. For example, when a bone cement biocompatible filer material has hardened or set within the treatment area, or when a therapeutic biocompatible filler material has provided a therapeutic effect to a tissue within the treatment area.

The illustrative method 400 further comprises removing the expansible body from the treatment area along a path established by the first hollow member, as shown in box 475. For example, the expansible body may be disposed at a distal end of an elongate member (as shown in Figures 1 and 2), and a user may grasp and remove the elongate member from the first hollow member, thereby removing the expansible body from the treatment area through the first hollow member. In other treatment methods, the expansible body may be separable from an elongated member used to insert it into the treatment area, and may be left implanted in either an expanded or collapsed state within the treatment area while the elongated member is removed through the first hollow member.

The method 400 shown in Figure 8 finally comprises inserting the biocompatible filler material into the treatment area through the first hollow member, as shown in box 485. The biocompatible filler material (such as a bone cement) may be inserted into the treatment area to fill a space formerly occupied by the expansible body. In one treatment method, the biocompatible filler material may inserted into the treatment area through the first hollow member after the biocompatible filler material inserted into the treatment area through the second hollow member has hardened. The biocompatible filler material, may remain in the treatment area in the described treatment method, and may provide dimensional stability to the treatment area after the expansible body has been removed.

In one treatment method, the biocompatible filler material inserted into the treatment area through the first hollow member may differ in composition, effect, temperature, state, or some other property from the biocompatible filler material inserted into the treatment area through the second hollow member. In yet another treatment method, another surgical tool, such as a scope, may also be inserted into the treatment area through the first or second hollow members.

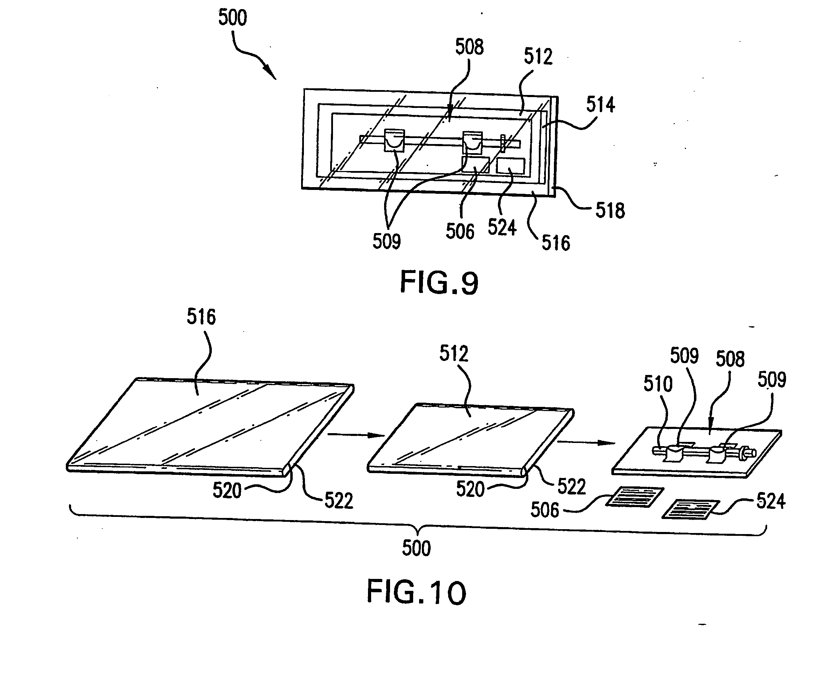

Referring now to Figures 9 and 10, a plan view and an exploded perspective view, respectively, of a sterile kit to store a cavity-forming tool according to one embodiment of the present invention is shown. At least some parts of a system according to one embodiment of the present invention (such as the system 210 described above) may be packaged in a sterile kit 500 as shown in Figures 9 and 10 prior to deployment in a bone or other tissue. In one such embodiment, the tool may comprise a single use tool.

As shown in Figures 9 and 10, the kit 500 comprises an interior tray 508. The tray 508 holds the system (generically designated 510) in a lay-flat, straightened condition during sterilization and storage prior to its first use. The tray 508 can be formed, for example, from die cut cardboard or thermoformed plastic material. The tray 508 comprises one or more spaced apart tabs 509, which hold the system 510 in the desired lay-flat, straightened condition.

The kit 500 comprises an inner wrap 512 that, in the embodiment shown, is peripherally sealed by heat or the like, to enclose the tray 508 from contact with the outside environment. One end of the inner wrap 512 comprises a conventional peal-away seal 514 (see Figure 10), to provide quick access to the tray 508 upon use, which may occur in a sterile environment, such as within an operating room.

The kit 500 shown also comprises an outer wrap 516, which is also peripherally sealed by heat or the like, to enclose the inner wrap 512. One end of the outer wrap 516 comprises a conventional peal-away seal 518 (see Figure 10), to provide access to the inner wrap 512, which can be removed from the outer wrap 516 in anticipation of imminent use of the system 510, without compromising sterility of the system 510 itself.

Both inner and outer wraps 512 and 516 (see Figure. 10) comprise a peripherally sealed top sheet 520 and bottom sheet 522. In the illustrated embodiment, the top sheet 520 is made of transparent plastic film, like polyethylene or MYLAR^{™} material, to allow visual identification of the contents of the kit 500. The bottom sheet 522 may be made from a material permeable to ethylene oxide sterilization gas, e.g., TYVEC^{™} plastic material (available from DuPont®).

In the embodiment shown in Figures 9 and 10, the sterile kit 500 also carries a label or insert 506, which comprises the statement "For Single Patient Use Only" (or comparable language) to affirmatively caution against reuse of the contents of the kit 500. The label 506 also may affirmatively instruct against resterilization of the system 510. The label 506 also may instruct the physician or user to dispose of the system 510 and the entire contents of the kit 500 upon use in accordance with applicable biological waste procedures. The presence of the system 510 packaged in the kit 500 verifies to the physician or user that the system 510 is sterile and has not been subjected to prior use. The physician or user is thereby assured that the system 510 meets established performance and sterility specifications, and will comprise the expected configuration when used.

The kit 500 also may comprise directions for use 524, which may instruct the physician regarding the use of the system 510. For example, the directions 524 may instruct the physician to deploy, manipulate, and adjust the system 510 inside a bone or other tissue to provide, adjust, or maintain a cavity. The directions 524 can also instruct the physician to fill the cavity with a biocompatible filler material, e.g., bone cement, allograft material, synthetic bone substitute, a medication, or a flowable material that sets to a hardened condition before, during, or after the system 510 has provided, adjusted, or maintained the cavity.

The foregoing description of illustrative embodiments of the invention has been presented only for the purpose of illustration and description and is not intended to be exhaustive or to limit the invention to the precise forms disclosed. Numerous modifications and adaptations thereof will be apparent to those skilled in the art without departing from the scope of the present invention as defined in the claims.

Furthermore, where methods and steps described above indicate certain events occurring in certain orders, those of ordinary skill in the art having the benefit of this disclosure would recognize that the ordering of certain steps may be modified and that such modifications are in accordance with the variations of the invention. Additionally, certain of the steps may be performed concurrently in a parallel process when possible, as well as performed sequentially as described above. Thus, the breadth and scope of the invention should not be limited by any of the above-described embodiments, but should be defined only in accordance with the following claims.

## Claims

1. A system (10, 210, 310) for inserting biocompatible filler materials into an interior body region, comprising:
an elongate member (40, 240, 340) comprising a distal end (46, 246);
a first expansible body (50, 250, 350) comprising a first body material, the first expansible body disposed at the distal end of the elongate member and configured to be maneuverable along a path established by a hollow member (20, 220, 320); and
a second expansible body (280, 380) disposed within the first expansible body and
comprising a second body material;
the first and second expansible bodies being expandable to provide a first cavity within the interior body region;
**characterised in that** the first expansible body is substantially resistant to at least one of a biocompatible filler material and a chemical component of the biocompatible filler material, and **in that** the system comprises a third expansible body (382) which is expandable to provide a second cavity within the interior body region.

2. The system of claim 1, wherein the first expansible body (50, 250, 350) is coupled to the second expansible body.

3. The system of claim 1 or 2, wherein the first expansible body (50, 250, 350) comprises a sheath surrounding at least a portion of the second expansible body (280, 380).

4. The system of claim 1 or 2, wherein the first expansible body (50, 250, 350) comprises a coating disposed on at least a portion of the expansible body (280, 380).

5. The system of any preceding claim, further comprising:
the hollow member (20, 220, 320).

6. The system of any preceding claim, wherein the second body material comprises a compliant material.

7. The system of any preceding claim, wherein the second body material comprises a polyurethane-based material.

8. The system of any preceding claim, wherein the second expansible body (280, 380) is configured to have a predetermined expanded dimension.

9. The system of claim 8, wherein at least the second expansible body (280, 380) is selected from a plurality of expansible bodies, each configured to have a unique predetermined expanded dimension.

10. The system of any preceding claim, wherein the first expansible body (50, 250, 350) is configured to have a predetermined expanded dimension.

11. The system of claim 10, wherein at least the first expansible body (50, 250, 350) is selected from a plurality of first expansible bodies, each configured to have a unique predetermined expanded dimension.

12. The system of claim 10 or 11, wherein the distal end of the elongate member (40, 240, 340) comprises a first distal end (46), the hollow member (20, 220, 320) comprises a second distal end (24, 224, 324), and the first expansible body (50, 250, 350) is configured to be expanded from a first dimension to the predetermined expanded dimension when extended to a point beyond the second distal end.

13. The system of claim 12, wherein at least the first expansible body (50, 250, 350) is configured to be contracted from the predetermined expanded dimension to the first dimension when brought within the second distal end (24, 224, 324) from the point beyond the second distal end.

14. The system of any preceding claim, wherein the second expansible body (280, 380) is configured to maintain a dimension of a treatment area while at least a portion of the treatment area is filled with the biocompatible filler material.

15. The system of any preceding claim, wherein the first body material comprises a substantially non-compliant material.

16. The system of any preceding claim, wherein the first body material comprises a rupture-resistant material.

17. The system of any preceding claim, wherein the first body material is configured to substantially resist adhesion to the biocompatible filler material.

18. The system of any of claims 1-16, wherein the first body material is configured to substantially adhere to the biocompatible filler material.

19. The system of any preceding claim, wherein at least a portion of the first expansible body (50, 250, 350) comprises a therapeutic material configured to provide a therapeutic effect to a treatment area.

20. The system of any preceding claim, comprising the biocompatible filler material (386), wherein the biocompatible filler material comprises a therapeutic material.

21. The system of any preceding claim, wherein the second expansible body (280, 380) is configured to be expanded with at least one of the following: a gaseous material and a liquid material.

22. The system of any preceding claim, wherein the first expansible body (50, 250, 350) is configured to collapse as the second expansible body (280, 380) contracts.

23. The system of any preceding claim, wherein the elongate member (40, 240, 340) comprises a bore extending therethrough.

24. The system of claim 23, wherein the bore is configured to receive a surgical tool.

25. The system of any preceding claim, wherein the first body material is non-porous.

26. The system of any preceding claim, wherein the first expansible body (50, 250, 350) is configured to prevent contact between at least a portion of the second expansible body (280, 380) and the biocompatible filler material in use.

27. The system of any preceding claim, wherein the first expansible body (50, 250, 350) is configured to expand as the second expansible body (280, 380) expands.

28. The system of any of claims 1-26, wherein the first expansible body (50, 250, 350) may be at least partly expanded independent of the second expansible body (280, 380).

29. The system of claim 28, wherein the third expansible body (382) is disposed within a fourth expansible body comprising a body material substantially insoluble or resistant to a biocompatible filler material.

## Patentansprüche

1. Ein System (10, 210, 310) zum Einführen von biokompatiblen Füllmaterialien in eine innere Körperregion, umfassend:
ein längliches Element (40, 240, 340), das ein distales Ende (46, 246) umfasst;
einen ersten expandierbaren Körper (50, 250, 350), der ein erstes Körpermaterial umfasst, wobei der erste expandierbare Körper an dem distalen Ende des länglichen Elements angeordnet und konfiguriert ist,
entlang eines durch ein hohles Element (20, 220, 320) geschaffenen Wegs manövrierbar zu sein; und
einen zweiten expandierbaren Körper (280, 380), der in dem ersten expandierbaren Körper angeordnet ist und ein zweites Körpermaterial umfasst;
wobei der erste und zweite expandierbare Körper expandierbar ist, um einen ersten Hohlraum in der inneren Körperregion bereitzustellen;
**dadurch gekennzeichnet, dass** der erste expandierbare Körper gegenüber mindestens einem aus einem biokompatiblen Füllmaterial und einer chemischen Komponente des biokompatiblen Füllmaterials im Wesentlichen resistent ist, und dadurch, dass das System einen dritten expandierbaren Körper (382) umfasst, der expandierbar ist, um einen zweiten Hohlraum in der inneren Körperregion bereitzustellen.

2. Das System nach Anspruch 1, wobei der erste expandierbare Körper (50, 250, 350) mit dem zweiten expandierbaren Körper gekoppelt ist.

3. Das System nach Anspruch 1 oder 2, wobei der erste expandierbare Körper (50, 250, 350) eine Hülse umfasst, die mindestens einen Teil des zweiten expandierbaren Körpers (280, 380) umgibt.

4. Das System nach Anspruch 1 oder 2, wobei der erste expandierbare Körper (50, 250, 350) eine Beschichtung umfasst, die auf mindestens einem Teil des expandierbaren Körpers (280, 380) aufgebracht ist.

5. Das System nach einem vorstehenden Anspruch, ferner umfassend:
das hohle Element (20, 220, 320).

6. Das System nach einem vorstehenden Anspruch, wobei das zweite Körpermaterial ein nachgiebiges Material umfasst.

7. Das System nach einem vorstehenden Anspruch, wobei das zweite Körpermaterial ein auf Polyurethan basierendes Material umfasst.

8. Das System nach einem vorstehenden Anspruch, wobei der zweite expandierbare Körper (280, 380) konfiguriert ist, eine vorbestimmte expandierte Abmessung aufzuweisen.

9. Das System nach Anspruch 8, wobei mindestens der zweite expandierbare Körper (280, 380) aus einer Vielzahl von expandierbaren Körpern ausgewählt ist, von denen jeder konfiguriert ist, eine einzigartige vorbestimmte expandierte Abmessung aufzuweisen.

10. Das System nach einem vorstehenden Anspruch, wobei der erste expandierbare Körper (50, 250, 350) konfiguriert ist, eine vorbestimmte expandierte Abmessung aufzuweisen.

11. Das System nach Anspruch 10, wobei mindestens der erste expandierbare Körper (50, 250, 350) aus einer Vielzahl von ersten expandierbaren Körpern ausgewählt ist, von denen jeder konfiguriert ist, eine einzigartige vorbestimmte expandierte Abmessung aufzuweisen.

12. Das System nach Anspruch 10 oder 11, wobei das distale Ende des länglichen Elements (40, 240, 340) ein erstes distales Ende (46) umfasst, das hohle Element (20, 220, 320) ein zweites distales Ende (24, 224, 324) umfasst und der erste expandierbare Körper (50, 250, 350) konfiguriert ist, von einer ersten Abmessung zu der vorbestimmten expandierten Abmessung expandiert zu werden, wenn er zu einem Punkt über das zweite distale Ende hinaus expandiert wird.

13. Das System nach Anspruch 12, wobei mindestens der erste expandierbare Körper (50, 250, 350) konfiguriert ist, von der vorbestimmten expandierten Abmessung zu der ersten Abmessung kontrahiert zu werden, wenn er in das zweite distale Ende (24, 224, 324) von dem Punkt über das zweite distale Ende hinaus gebracht wird.

14. Das System nach einem vorstehenden Anspruch, wobei der zweite expandierbare Körper (280, 380) konfiguriert ist, eine Abmessung eines Behandlungsbereichs zu halten, während mindestens ein Teil des Behandlungsbereichs mit dem biokompatiblen Füllmaterial gefüllt wird.

15. Das System nach einem vorstehenden Anspruch, wobei das erste Körpermaterial ein im Wesentlichen nicht-nachgiebiges Material umfasst.

16. Das System nach einem vorstehenden Anspruch, wobei das erste Körpermaterial ein reißfestes Material umfasst.

17. Das System nach einem vorstehenden Anspruch, wobei das erste Körpermaterial konfiguriert ist, einer Adhäsion an das biokompatible Füllmaterial im Wesentlichen zu widerstehen.

18. Das System nach einem der Ansprüche 1-16, wobei das erste Körpermaterial konfiguriert ist, im Wesentlichen an dem biokompatiblen Füllmaterial zu haften.

19. Das System nach einem vorstehenden Anspruch, wobei mindestens ein Teil des ersten expandierbaren Körpers (50, 250, 350) ein therapeutisches Material umfasst, das konfiguriert ist, eine therapeutische Wirkung für einen Behandlungsbereich bereitzustellen.

20. Das System nach einem vorstehenden Anspruch, das das biokompatible Füllmaterial (386) umfasst, wobei das biokompatible Füllmaterial ein therapeutisches Material umfasst.

21. Das System nach einem vorstehenden Anspruch, wobei der zweite expandierbare Körper (280, 380) konfiguriert ist, mit mindestens einem der folgenden expandiert zu werden: einem gasförmigen Material und einem flüssigen Material.

22. Das System nach einem vorstehenden Anspruch, wobei der erste expandierbare Körper (50, 250, 350) konfiguriert ist, zu kollabieren während der zweite expandierbare Körper (280, 380) kontrahiert.

23. Das System nach einem vorstehenden Anspruch, wobei das längliche Element (40, 240, 340) ein Bohrloch umfasst, das sich dorthindurch erstreckt.

24. Das System nach Anspruch 23, wobei das Bohrloch konfiguriert ist, ein chirurgisches Werkzeug aufzunehmen.

25. Das System nach einem vorstehenden Anspruch, wobei das erste Körpermaterial nicht-porös ist.

26. Das System nach einem vorstehenden Anspruch, wobei der erste expandierbare Körper (50, 250, 350) konfiguriert ist, bei Gebrauch einen Kontakt zwischen mindestens einem Teil des zweiten expandierbaren Körpers (280, 380) und dem biokompatiblen Füllmaterial zu verhindern.

27. Das System nach einem vorstehenden Anspruch, wobei der erste expandierbare Körper (50, 250, 350) konfiguriert ist, zu expandieren während der zweite expandierbare Körper (280, 380) expandiert.

28. Das System nach einem der Ansprüche 1-26, wobei der erste expandierbare Körper (50, 250, 350) mindestens zum Teil expandiert werden kann, und zwar unabhängig von dem zweiten expandierbaren Körper (280, 380).

29. Das System nach Anspruch 28, wobei der dritte expandierbare Körper (382) in einem vierten expandierbaren Körper angeordnet ist, der ein Körpermaterial umfasst, das gegenüber einem biokompatiblen Füllmaterial im Wesentlichen unlöslich oder resistent ist.

## Revendications

1. Système (10, 210, 310) d'insertion de matières de remplissage biocompatibles dans une région corporelle intérieure, comprenant :
un élément allongé (40, 240, 340) comprenant une extrémité distale (46, 246) ;
un premier corps expansible (50, 250, 350) comprenant une première matière de corps, le premier corps expansible étant disposé à l'extrémité distale de l'élément allongé et
configuré pour être manoeuvrable le long d'un trajet établi par un élément creux (20, 220, 320) ; et
un deuxième corps expansible (280, 380) disposé à l'intérieur du premier corps expansible et comprenant une deuxième matière de corps ;
les premier et deuxième corps expansibles étant aptes à s'étendre pour former une première cavité à l'intérieur de la région corporelle intérieure ;
**caractérisé par le fait que** le premier corps expansible est sensiblement résistant à au moins l'un d'une matière de remplissage biocompatible et d'un composant chimique de la matière de remplissage biocompatible, et **par le fait que** le système comprend un troisième corps expansible (382) qui est apte à s'étendre pour former une seconde cavité à l'intérieur de la région corporelle intérieure.

2. Système selon la revendication 1, dans lequel le premier corps expansible (50, 250, 350) est couplé au deuxième corps expansible.

3. Système selon la revendication 1 ou 2, dans lequel le premier corps expansible (50, 250, 350) comprend une gaine entourant au moins une partie du deuxième corps expansible (280, 380).

4. Système selon la revendication 1 ou 2, dans lequel le premier corps expansible (50, 250, 350) comprend un revêtement disposé sur au moins une partie du corps expansible (280, 380).

5. Système selon l'une quelconque des revendications précédentes, comprenant en outre :
l'élément creux (20, 220, 320).

6. Système selon l'une quelconque des revendications précédentes, dans lequel la deuxième matière de corps comprend une matière souple.

7. Système selon l'une quelconque des revendications précédentes, dans lequel la deuxième matière de corps comprend une matière à base de polyuréthane.

8. Système selon l'une quelconque des revendications précédentes, dans lequel le deuxième corps expansible (280, 380) est configuré pour avoir une dimension étendue prédéterminée.

9. Système selon la revendication 8, dans lequel au moins le deuxième corps expansible (280, 380) est choisi parmi plusieurs corps expansibles, chacun étant configuré pour avoir une dimension étendue prédéterminée unique.

10. Système selon l'une quelconque des revendications précédentes, dans lequel le premier corps expansible (50, 250, 350) est configuré pour avoir une dimension étendue prédéterminée.

11. Système selon la revendication 10, dans lequel au moins le premier corps expansible (50, 250, 350) est choisi parmi plusieurs premiers corps expansibles, chacun étant configuré pour avoir une dimension étendue prédéterminée unique.

12. Système selon la revendication 10 ou 11, dans lequel l'extrémité distale de l'élément allongé (40, 240, 340) comprend une première extrémité distale (46), l'élément creux (20, 220, 320) comprend une seconde extrémité distale (24, 224, 324) et le premier corps expansible (50, 250, 350) est configuré pour être étendu d'une première dimension à la dimension étendue prédéterminée lorsqu'il est amené à s'étendre jusqu'à un point au-delà de la seconde extrémité distale.

13. Système selon la revendication 12, dans lequel au moins le premier corps expansible (50, 250, 350) est configuré pour être contracté de la dimension étendue prédéterminée à la première dimension lorsqu'il est amené à l'intérieur de la seconde extrémité distale (24, 224, 324) à partir du point au-delà de la seconde extrémité distale.

14. Système selon l'une quelconque des revendications précédentes, dans lequel le deuxième corps expansible (280, 380) est configuré pour maintenir une dimension d'une région de traitement tandis qu'au moins une partie de la région de traitement est remplie de la matière de remplissage biocompatible.

15. Système selon l'une quelconque des revendications précédentes, dans lequel la première matière de corps comprend une matière sensiblement non souple.

16. Système selon l'une quelconque des revendications précédentes, dans lequel la première matière de corps comprend une matière résistant à la rupture.

17. Système selon l'une quelconque des revendications précédentes, dans lequel la première matière de corps est configurée pour résister sensiblement à une adhésion à la matière de remplissage biocompatible.

18. Système selon l'une quelconque des revendications 1 à 16, dans lequel la première matière de corps est configurée pour adhérer sensiblement à la matière de remplissage biocompatible.

19. Système selon l'une quelconque des revendications précédentes, dans lequel au moins une partie du premier corps expansible (50, 250, 350) comprend une matière thérapeutique configurée pour apporter un effet thérapeutique à une région de traitement.

20. Système selon l'une quelconque des revendications précédentes, comprenant la matière de remplissage biocompatible (386), la matière de remplissage biocompatible comprenant une matière thérapeutique.

21. Système selon l'une quelconque des revendications précédentes, dans lequel le deuxième corps expansible (280, 380) est configuré pour être étendue avec au moins l'un des suivants : une matière gazeuse et une matière liquide.

22. Système selon l'une quelconque des revendications précédentes, dans lequel le premier corps expansible (50, 250, 350) est configuré pour s'affaisser au fur et à mesure que le deuxième corps expansible (280, 380) se contracte.

23. Système selon l'une quelconque des revendications précédentes, dans lequel l'élément allongé (40, 240, 340) comprend un alésage s'étendant à travers celui-ci.

24. Système selon la revendication 23, dans lequel l'alésage est configuré pour recevoir un outil chirurgical.

25. Système selon l'une quelconque des revendications précédentes, dans lequel la première matière de corps est non poreuse.

26. Système selon l'une quelconque des revendications précédentes, dans lequel le premier corps expansible (50, 250, 350) est configuré pour empêcher en utilisation un contact entre au moins une partie du deuxième corps expansible (280, 380) et la matière de remplissage biocompatible.

27. Système selon l'une quelconque des revendications précédentes, dans lequel le premier corps expansible (50, 250, 350) est configuré pour s'étendre lorsque le deuxième corps expansible (280, 380) s'étend.

28. Système selon l'une quelconque des revendications 1 à 26, dans lequel le premier corps expansible (50, 250, 350) peut être au moins partiellement étendu indépendamment du deuxième corps expansible (280, 380).

29. Système selon la revendication 28, dans lequel le troisième corps expansible (382) est disposé à l'intérieur d'un quatrième corps expansible comprenant une matière de corps sensiblement insoluble dans ou résistant à une matière de remplissage biocompatible.
